(19) Europäisches Patentamt
European Patent Office
Office européen des brevets

(11) Publication number: **0 084 377**
**B1**

(12) **EUROPEAN PATENT SPECIFICATION**

(45) Date of publication of patent specification: **27.12.85**

(51) Int. Cl.⁴: **C 07 D 495/04** // A61K31/415 ,(C07D495/04, 333:00, 235:00)

(21) Application number: **83100397.5**

(22) Date of filing: **18.01.83**

(54) **Process for preparing biotin.**

(30) Priority: **19.01.82 JP 7253/82**

(43) Date of publication of application:
**27.07.83 Bulletin 83/30**

(45) Publication of the grant of the patent:
**27.12.85 Bulletin 85/52**

(84) Designated Contracting States:
**CH DE FR IT LI**

(56) References cited:

**CHEMICAL ABSTRACTS, vol. 70, no. 5, 3rd February 1969, page 1990, no. 19984r, Columbus, Ohio, USA ISAKA et al.: "Biotin synthesis. IV. a new synthesis of biotin by grignard reaction of 1,4-butylenedimagnesium halide"**

(73) Proprietor: **SUMITOMO CHEMICAL COMPANY, LIMITED**
**15 Kitahama 5-chome Higashi-ku**
**Osaka-shi Osaka 541 (JP)**

(72) Inventor: **Ohashi, Naohito**
**4-2-406, Ryodo-cho**
**Nishinomiya Hyogo (JP)**
Inventor: **Ikeda, Takaharu**
**10-13-109, Makitsukadai 1-chome**
**Sakai Osaka (JP)**
Inventor: **Shimago, Kozo**
**10-4-412, Sonehigashimachi 2-chome**
**Toyonaka Osaka (JP)**
Inventor: **Takahashi, Takeo**
**3-8-10, Satsukidai Takarazuka**
**Hyogo (JP)**
Inventor: **Ishizumi, Kikuo**
**2-16-10, Uenonishi**
**Toyonaka Osaka (JP)**

(74) Representative: **Vossius Vossius Tauchner Heunemann Rauh**
**Siebertstrasse 4 P.O. Box 86 07 67**
**D-8000 München 86 (DE)**

**EP 0 084 377 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to a process for preparing biotin. More particularly, it relates to an industrially advantageous process for preparing biotin.

The process of this invention comprises (a) reacting the compound of the formula:

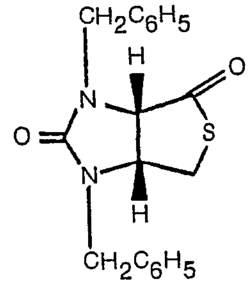

(I)

with a compound of the formula:

$$(C_6H_5)_3P^+(CH_2)_4COOR_1.X^- \qquad (II)$$

wherein $R_1$ is hydrogen or $C_1$—$C_4$ alkyl and X is halogen in the presence of a base to give a compound of the formula:

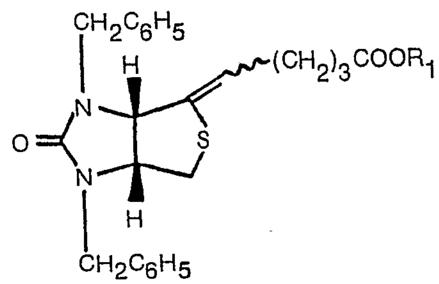

(III)

wherein $R_1$ is as defined above, (b) subjecting the latter to reduction to give a compound of the formula:

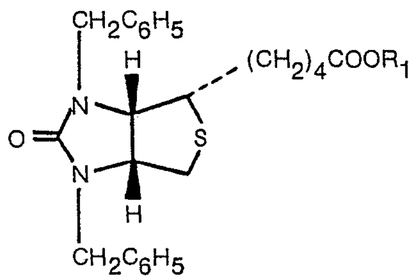

(IV)

wherein $R_1$ is as defined above and (c) subjecting the latter to debenzylation to give a compound of the formula:

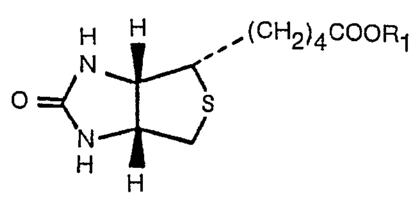

(V)

wherein $R_1$ is as defined above, in case of $R_1$ being $C_1$—$C_4$ alkyl, optionally with previous or subsequent hydrolysis of the ester to give the corresponding acid.

In the above significances, the term "$C_1$—$C_4$ alkyl" includes methyl, ethyl, n-propyl, n-butyl, sec-butyl, t-butyl, etc. The term "halogen" is intended to mean chlorine, bromine or iodine.

As well known, biotin (V: $R_1$ = hydrogen) is a valuable pharmaceutical substance exerting a preventive and therapeutic effect on dermatoses as well as a growth accelerative effect.

Various processes are known for preparing biotin, among which the one proceeding through the compound (I) is the most advantageous from the industrial viewpoint. In this process, it is necessary to introduce a $C_5$ side chain having a carboxyl group at the terminal position into the compound (I). In order to realize such introduction, there has been adopted a procedure wherein a $C_3$ chain is first introduced and then a $C_2$ chain is introduced (cf. Japanese Patent Publns. Nos. 1420/1952 and 4481/1953) or a procedure wherein a $C_4$ chain is first introduced and then a $C_1$ chain is introduced (cf. Japanese Patent Publn. No. 3580/1971).

As a result of extensive study, it has been found that the reaction of the compound (I) with the compound (II) in the presence of a base can enable the introduction of the said $C_5$ side chain into the compound (I) in a single step. On the basis of this finding, there has been completed an industrially advantageous process for producing biotin through the compound (I).

This process i.e., the reaction between the compound (I) and the compound (II) in the presence of a base to give the compound (III) is a kind of Wittig reaction.

It is generally known that a normal Wittig reaction does not proceed between the molecules of a thioester (cf. Modern Synthetic Reactions, 2nd Ed., p. 692 (1972); Carbon-Carbon Bond Formation, *1*, p. 362 (1973)); Organophosphorous Reagents in Organic Synthesis, p. 212 (1979); Organic Reactions, *14*, p. 292 (1965)). Contrary to such general knowledge, it was unexpectedly found that the Wittig reaction can proceed smoothly and successfully between the compound (I) and the compound (II) to give the compound (III). Thus, the introduction of the $C_5$ side chain having a terminal carboxy group into the compound (I) can be realized in one step.

As stated above, the process of this invention comprises the steps of (a) reaction of the compound (I) with the compound (II) to give the compound (III), (b) reduction of the compound (III) to the compound (IV) and (c) debenzylation of the compound (IV) to the compound (V), optionally with the step of previous or subsequent hydrolysis of the compound (IV) or (V) wherein $R_1$ is alkyl, to the corresponding compound (IV) or (V) wherein $R_1$ is hydrogen.

The above steps are explained in detail as follows: the first step (a) is concerned with the reaction between the compound (I) and the compound (II) in the presence of a base.

Examples of suitable bases are alkyl alkali metals (e.g. n-butyl lithium, sec-butyl lithium), carbanions derived from alkali metal hydrides (e.g. lithium hydride, sodium hydride, potassium hydride) and dimethylsulfoxides such as lithium methylsulfinylcarbanion, sodium methylsulfinylcarbanion and potassium methylsulfinylcarbanion, alkali metal alkoxides (e.g. sodium methoxide, potassium t-butoxide), alkali metal amides (e.g. lithium amide, sodium amide, potassium amide), alkali metal alkylamides (e.g. lithium diisopropylamide), alkali metal hexamethyldisilazanes (e.g. lithium hexamethyldisilazane, sodium hexamethyldisilazane), alkali metal hydroxides (e.g. lithium hydroxide, sodium hydroxide, potassium hydroxide) and alkali metal carbonates (e.g. sodium carbonate, potassium carbonate).

The molar proportion of the compound (II) to the compound (I) is usually from 1 to 5, preferably from 1.5 to 2.5. The base may be used in a molar amount of 2—3 to the compound (II) wherein $R_1$ is hydrogen and of 1—2 to the compound (II) wherein $R_1$ is $C_1$—$C_4$ alkyl.

Any solvent that does not produce any adverse effect on the course of the reaction may be used. Preferred examples of the solvent are ethers (e.g. tetrahydrofuran, dioxane, diethyl ether), aromatic hydrocarbons (e.g. benzene, toluene), halogenated alkanes (e.g. dichloromethane, chloroform, dichloroethane) and dimethylsulfoxide.

Alcohols (e.g. methanol, t-butanol) may be also used unless they react with the base. When, for instance, the base is an alkyl alkali metal, a carbanion of dimethylsulfoxide or an alkali metal hexamethyldisilazane, the use of alcohols should be avoided, because those bases react with alcohols. The reaction temperature may be usually from −50 to 100°C, preferably from −20 to 70°C.

In carrying out the reaction, there is no fixed order for charging the reactants into a reactor. For instance, the compound (II) may be previously reacted with the base, followed by introduction of the compound (I) into the reaction mixture. Further, for instance, the compound (I), the compound (II) and the base may be introduced into the reactor simultaneously and then subjected to the reaction. When the base is reacted with the thioester portion of the compound (I), the former operation is favorable. When the compound (II) has an alkoxycarbonyl group, it is desired to avoid the use of any base which is apt to be reactive with an ester. Alkyl alkali metals, carbanions of dimethylsulfoxide, alkali metal alkoxides and alkali metal hydroxides are typical examples of the bases as readily reactive with an ester.

After the reaction is completed, the reaction mixture is preferably acidified, followed by heating.

The second step (b) is concerned with the reduction of the compound (III) to the compound (IV).

The reduction may be accomplished by a per se conventional catalytic reduction procedure, e.g. reacting the compound with hydrogen in the presence of a catalyst. Examples of the catalyst are palladium-carbon, palladium oxide, sulfur-resistant nickel, platinum oxide, rutenium, rhodium, etc. Any solvent that does not produce any adverse effect on the course of the reaction may be used. Preferred examples of the solvent are aromatic hydrocarbons (e.g. benzene, toluene), alcohols (e.g. methanol, ethanol, t-butanol), esters (e.g. ethyl acetate, butyl acetate, ethers (e.g. tetrahydrofuran, dioxane), water, acetic acid, etc. The reaction can proceed at room temperature but may be accelerated by elevating the temperature to 40—150°C. Likewise, the reaction is preferably carried out under a hydrogen pressure of 3 to 100 atm, although an ordinary pressure may be adopted.

The third step (c) is concerned with debenzylation of the compound (IV) to the compound (V).

The debenzylation may be achieved by a per se conventional procedure as adopted in the synthesis of biotin (cf. Japanese Patent Publications Nos. 1420/1952, 31669/1970 and 27279/1978). As the result, there is usually obtained the compound (V) wherein $R_1$ corresponds to the one present in the starting compound (IV).

When the product is the compound (V) wherein $R_1$ is alkyl, it may be hydrolyzed by treatment with a base (e.g. sodium hydroxide, potassium hydroxide). Alternatively, such hydrolysis may be applied to the compound (IV) wherein $R_1$ is alkyl so that the compound (V) wherein $R_1$ is hydrogen is obtained as the product. This product may then be subjected to debenzylation in the manner mentioned above.

As an advantageous procedure for debenzylation, the compound (IV) wherein $R_1$ is alkyl may be treated with a mineral acid (e.g. hydrobromic acid) in an aqueous medium while heating (e.g. 30 to 90°C), whereby debenzylation and hydrolysis proceed simultaneously to afford the compound (V) wherein $R_1$ is hydrogen (i.e. biotin) in one step.

In the process of the present invention, the optical property of the compound (V) corresponds to that of the starting compound (I). When, for instance, the starting compound (I) is in a racemic form, the produced compound (V) is also in a racemic form. When, for instance, the starting compound (I) is in an optically active form, the produced compound (V) is also in an optically active form since no racemization takes place at any of the steps making up the process of the invention.

Practical and presently preferred embodiments of the invention are illustratively shown in the following Examples where % is by weight unless otherwise indicated.

Example 1

(A) 50% Sodium hydride (1.7 g) was washed with n-hexane (10 ml) under nitrogen stream, followed by addition of dimethylsulfoxide (17 ml) thereto. The resultant mixture was stirred at 70°C until generation of hydrogen gas stopped. After allowing it to cool to room temperature, a solution of (4-carboxybutyl)triphenylphosphonium bromide (8.0 g) in dimethylsulfoxide (10 ml) was added thereto, and the mixture was stirred for 15 minutes to prepare the Wittig reagent.

Into a separate flask were charged d-hexahydrothieno-[3,4-d]-imidazole-2,4-dione (3.0 g; M.P., 122—123°C; $[\alpha]_D^{22}$ = +90.7° (c = 1.00, chloroform)), dimethylsulfoxide (10 ml) and toluene (2 ml), and the above prepared Wittig reagent was dropwise added thereto. The resultant mixture was stirred for 20 minutes. Ice water (5 g) and conc. hydrochloric acid (10 ml) were added to the mixture while stirring, followed by further addition of ice (100 g). An oily substance was separated, water (50 ml), benzene (100 ml) and ethyl acetate (50 ml) were added thereto in this order and the mixture was stirred at 50—60°C for 1.5 hours. The organic layer was dried over anhydrous magnesium sulfate and filtered. The filtrate was concentrated under reduced pressure and subjected to silica gel column chromatography using a mixture of benzene and ethyl acetate (4:1) as an eluting solvent to give 3.1 g of d-hexahydro-2-oxo-1,3-dibenzylthieno[3,4-d]imidazol-4-ylidenepentanoic acid. M.P., 81—83°C. $[\alpha]_D^{22}$ = +217° (c = 1.01, methanol).

(B) (4-Carboxybutyl)triphenylphosphonium bromide (8.0 g) was added to tetrahydrofuran (50 ml) while stirring under nitrogen stream, followed by dropwise addition of a 2.34 N hexane solution of n-butyl lithium (15 ml). The resultant mixture was stirred for 30 minutes and then ice-cooled. A solution of d-hexahydrothieno[3,4-d]imidazole-2,4-dione (3.0 g; M.P., 122—123°C; $[\alpha]_D^{20}$ = +90.7° (c = 1.00, chloroform)) in tetrahydrofuran (20 ml) was dropwise added to the mixture in 20 minutes and stirred for 1 hour. Ice water (10 g) and conc. hydrochloric acid (10 ml) were added to the reaction mixture while stirring, followed by further addition of ice (10 g). The resultant mixture was extracted with ethyl acetate, and the extract was dried over anhydrous magnesium sulfate and filtered. The filtrate was concentrated under reduced pressure, and the condensate was combined with benzene (100 ml), kept at 50°C for 2 hours and again concentrated. The resulting condensate was subjected to silica gel column chromatography using a mixture of benzene and ethyl acetate (5:1) as an eluting solvent to give 2.4 g of d-hexahydro-2-oxo-1,3-dibenzylthieno[3,4-d]imidazol-4-ylidenepentanoic acid. M.P., 80—82°C. $[\alpha]_D^{22}$ = +216° (c = 1.03, methanol).

Example 2

(A) d-Hexahydro-2-oxo-1,3-dibenzylthieno[3,4-d]imidazol-4-ylidenepentanoic acid (400 mg; M.P., 81—83°C; $[\alpha]_D^{22}$ = +217° (c = 1.01, methanol)) was dissolved in 2-propanol (20 ml), and 10% palladium carbon (100 mg) was added thereto. Reductive hydrogenation was effected at 50°C for 18 hours under a hydrogen pressure of 10 kg/cm$^2$. The resultant mixture was subjected to celite filtration and concentrated under reduced pressure to give d-hexahydro-2-oxo-1,3-dibenzylthieno[3,4-d]imidazole-4-pentanoic acid in a quantitative yield. M.P., 89—91°C. $[\alpha]_D^{22}$ = −24.0° (c = 1.01, methanol).

(B) d-Hexahydro-2-oxo-1,3-dibenzylthieno[3,4-d]imidazol-4-ylidenepentanoic acid (400 mg; M.P., 81—83°C; $[\alpha]_D^{22}$ = +217° (c = 1.01, methanol)) was dissolved in 2-propanol (20 ml), and palladium oxide (100 mg) was added thereto. Reductive hydrogenation was effected at room temperature under atmospheric pressure while stirring for 3 days. The resultant mixture was subjected to celite filtration and concentrated under reduced pressure to give d-hexahydro-2-oxo-1,3-dibenzylthieno[3,4-d]imidazole-4-pentanoic acid in a quantitative yield. M.P., 89—91°C. $[\alpha]_D^{22}$ = −24.0° (c = 1.00, methanol).

Example 3

d-Hexahydro-2-oxo-1,3-dibenzylthieno[3,4-d]imidazole-4-pentanoic acid (1.0 g; M.P., 89—91°C; $[\alpha]_D^{22} = -24.0°$ (c = 1.01, methanol)) was combined with methanesulfonic acid (10 g), and the resultant mixture was stirred at 140°C for 6 hours under heating. The reaction mixture was allowed to cool and dropwise added to water (50 ml) while stirring well to obtain d-biotin (0.48 g) as crude crystals. The crude crystals were recrystallized from water to give 0.42 g of d-biotin. M.P., 230—231°C. $[\alpha]_D^{20} = +91°$ (c = 1.00, 0.1% aqueous sodium hydroxide).

**Claims**

1. A process for preparing an intermediate for the synthesis of biotin which comprises the step of reacting the compound of the formula:

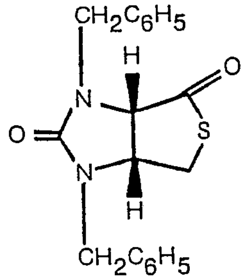

(I)

with a compound of the formula:

$$(C_6H_5)_3P^+(CH_2)_4COOR_1 \;.\; X^- \qquad (II)$$

wherein $R_1$ is a hydrogen atom or a $C_1$—$C_4$ alkyl group and X is a halogen atom in the presence of a base to give a compound of the formula:

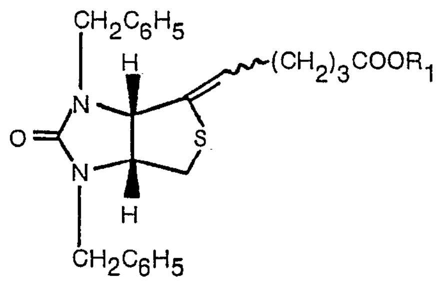

(III)

wherein $R_1$ is as defined above.

2. The process according to claim 1, wherein $R_1$ is a hydrogen atom.

3. The process according to claim 1, wherein the compound (I) is in a d-form.

4. The process according to claim 1, wherein the base is a compound of the formula:

$$CH_3—\overset{\overset{O}{\|}}{S}—CH_2^- \;.\; Y^+$$

wherein Y is a lithium atom, a sodium atom or a potassium atom.

5. The process according to claim 1, followed by subjecting the compound (III) to reduction to give a compound of the formula:

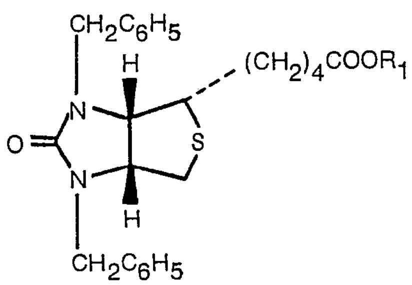

(IV)

wherein $R_1$ is as defined above.

6. The process according to claim 5, wherein the reduction is carried out by catalytic hydrogenation using palladium carbon, palladium oxide or sulfur-resistant nickel as the catalyst.

7. The process according to claim 5, followed by subjecting the compound (IV) to debenzylation to give a compound of the formula:

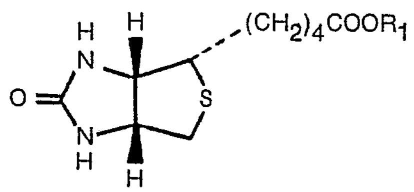

(V)

wherein $R_1$ is as defined above.

8. The process according to claim 7, wherein the debenzylation is carried out by treatment with methanesulfonic acid.

9. The process according to claim 7, in which the compound (IV) wherein $R_1$ is a $C_1$—$C_4$ alkyl group is first hydrolyzed to the compound (IV) wherein $R_1$ is a hydrogen atom and then the latter is subjected to debenzylation.

10. The process according to claim 7, in which the compound (IV) wherein $R_1$ is a $C_1$—$C_4$ alkyl group is first subjected to debenzylation to give the compound (V) wherein $R_1$ is a $C_1$—$C_4$ alkyl group and then the latter is hydrolyzed.

11. The process according to claim 7, in which the compound (IV) wherein $R_1$ is a $C_1$—$C_4$ alkyl group is subjected to debenzylation and hydrolysis simultaneously to give the compound (V) wherein $R_1$ is a hydrogen atom.

12. A process for preparing biotin or its ester which comprises the steps of (a) reacting the compound of the formula:

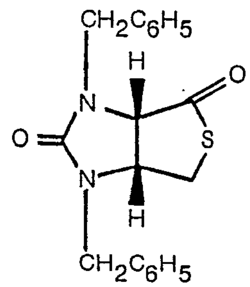

(I)

with a compound of the formula:

$$(C_6H_5)_3P^+(CH_2)_4COOR_1 \,.\, X^- \qquad (II)$$

wherein $R_1$ is a hydrogen atom or a $C_1$—$C_4$ alkyl group and X is a halogen atom in the presence of a base to give a compound of the formula:

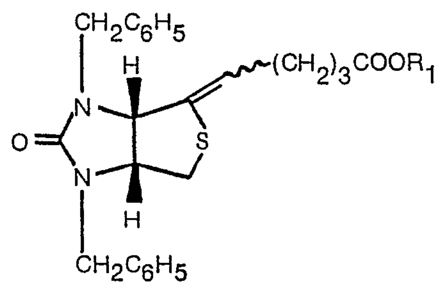

(III)

wherein $R_1$ is as defined above, (b) subjecting the latter to reduction to give a compound of the formula:

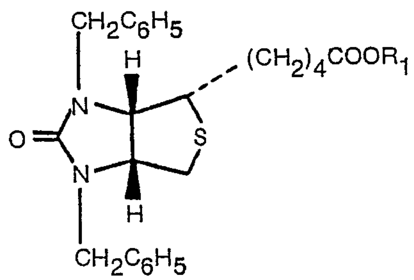

(IV)

wherein $R_1$ is as defined above and (c) subjecting the latter to debenzylation to give a compound of the formula:

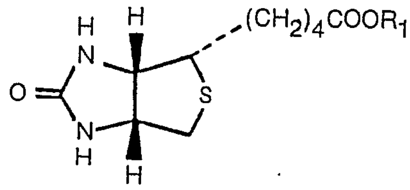

(V)

wherein $R_1$ is as defined above.

13. The process according to claim 12, wherein the compound (IV) wherein $R_1$ is a $C_1$—$C_4$ alkyl group is subjected to debenzylation, accompanied by previous hydrolysis, simultaneous hydrolysis or subsequent hydrolysis to give the compound (V) wherein $R_1$ is a hydrogen atom.

**Patentansprüche**

1. Verfahren zur Herstellung eines Zwischenproduktes zur Synthese von Biotin, dadurch gekennzeichnet, daß man in Gegenwart einer Base eine Verbindung der Formel (I):

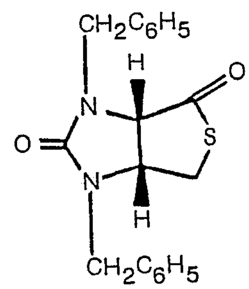

(I)

mit einer Verbindung der allgemeinen Formel (II) umsetzt:

$$(C_6H_5)_3P^+(CH_2)_4COOR_1 \,.\, X^- \qquad (II)$$

in der $R_1$ ein Wasserstoffatom oder eine $C_1$—$C_4$-Alkylgruppe und X ein Halogenatom ist und dabei eine Verbindung der allgemeinen Formel (III) erhält:

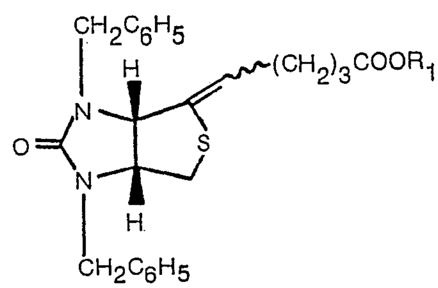

(III)

in der $R_1$ die vorstehend angegebene Bedeutung hat.

2. Verfahren nach Anspruch 1, in dem $R_1$ ein Wasserstoffatom ist.

3. Verfahren nach Anspruch 1, in dem die Verbindung der Formel (I) in der d-Form vorliegt.

4. Verfahren nach Anspruch 1, in dem die Base eine Verbindung der allgemeinen Formel:

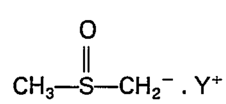

ist, in der Y ein Lithiumatom, ein Natrium- oder ein Kaliumatom ist.

5. Verfahren nach Anspruch 1, gefolgt von einer Reduktion der Verbindung der allgemeinen Formel (III) zu einer Verbindung der allgemeinen Formel (IV):

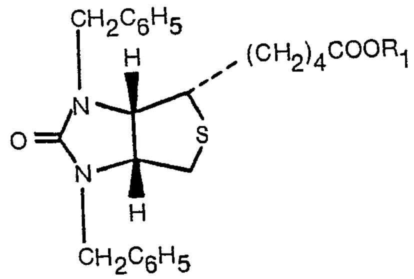

(IV)

in der $R_1$ die vorstehend angegebene Bedeutung hat.

6. Verfahren nach Anspruch 5, bei dem man die Reduktion durch katalytische Hydrierung unter Verwendung von Palladium-auf-Kohle, Palladiumoxid oder schwefelresistentem Nickel als Katalysator durchführt.

7. Verfahren nach Anspruch 5, gefolgt von einer Debenzylierung der Verbindung der allgemeinen Formel (IV) zu einer Verbindung der allgemeinen Formel (V):

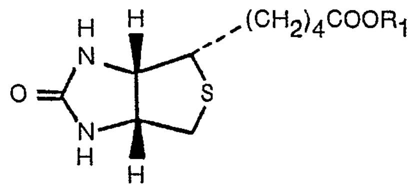

(V)

in der $R_1$ die vorstehend angegebene Bedeutung hat.

8. Verfahren nach Anspruch 7, bei dem man die Debenzylierung durch Behandlung mit Methansulfonsäure durchführt.

9. Verfahren nach Anspruch 7, bei dem man die Verbindung der allgemeinen Formel (IV), in der $R_1$ eine $C_1$—$C_4$-Alkylgruppe ist, zunächst zur Verbindung der allgemeinen Formel (IV) hydrolysiert, in der $R_1$ ein Wasserstoffatom ist, und danach debenzyliert.

10. Verfahren nach Anspruch 7, bei dem man die Verbindung der allgemeinen Formel (IV), in der $R_1$ eine $C_1$—$C_4$-Alkylgruppe ist, zunächst zu einer Verbindung der allgemeinen Formel (V) debenzyliert, in der $R_1$ eine $C_1$—$C_4$-Alkylgruppe ist, und man die letztere Verbindung danach hydrolysiert.

11. Verfahren nach Anspruch 7, bei dem man die Verbindung der allgemeinen Formel (IV), in der $R_1$ eine $C_1$—$C_4$-Alkylgruppe ist, gleichzeitig zu einer Verbindung der allgemeinen Formel (V), in der $R_1$ ein Wasserstoffatom ist, debenzyliert und hydrolysiert.

12. Verfahren zur Herstellung von Biotin oder seiner Ester, dadurch gekennzeichnet, daß man in Gegenwart einer Base (a) die Verbindung der Formel (I):

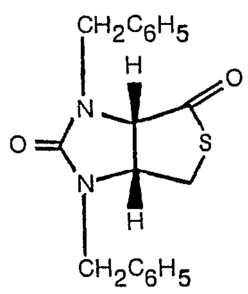

(I)

mit einer Verbindung der allgemeinen Formel (II) umsetzt:

$$(C_6H_5)_3P^+(CH_2)_4COOR_1 \,.\, X^- \qquad \text{(II)}$$

in der $R_1$ ein Wasserstoffatom oder eine $C_1$—$C_4$-Alkylgruppe und X ein Halogenatom ist und dabei eine Verbindung der allgemeinen Formel (III) erhält:

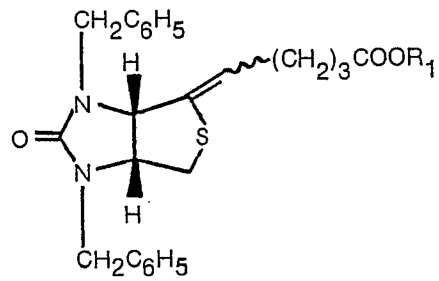

(III)

in der $R_1$ die vorstehend angegebene Bedeutung hat, (b) die erhaltene Verbindung zu einer Verbindung der allgemeinen Formel (IV) reduziert:

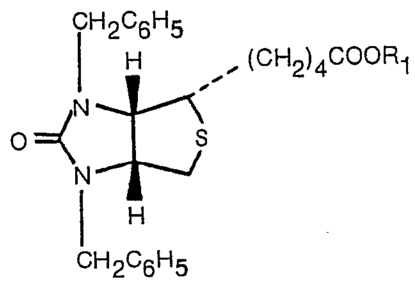

(IV)

in der $R_1$ die vorstehend angegebene Bedeutung hat und (c) die erhaltene Verbindung zu einer Verbindung der allgemeinen Formel (V) debenzyliert:

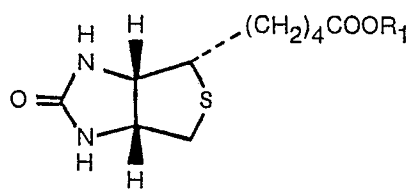

(V)

in der $R_1$ die vorstehend angegebene Bedeutung hat.

13. Verfahren nach Anspruch 12, bei dem man die Verbindung der allgemeinen Formel (IV), in der $R_1$ eine $C_1$—$C_4$-Alkylgruppe ist, debenzyliert, wobei man vorher hydrolysiert, gleichzeitig hydrolysiert oder nachfolgend hydrolysiert, und dabei eine Verbindung der allgemeinen Formel (V) erhält, in der $R_1$ ein Wasserstoffatom ist.

**Revendications**

1. Procédé de préparation d'un intermédiaire pour la synthèse de la biotine qui comprend l'étape de réaction du composé de formule:

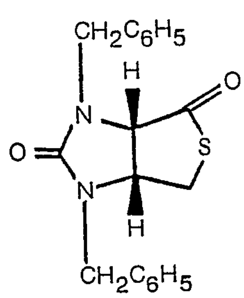

(I)

avec un composé de formule:

$$(C_6H_5)_3P^+(CH_2)_4COOR_1 \,.\, X^- \qquad (II)$$

dans laquelle $R_1$ est un atome d'hydrogène ou un groupe alkyle en $C_1$—$C_4$ et X est un atome d'halogène en présence d'une base pour donner un composé de formule:

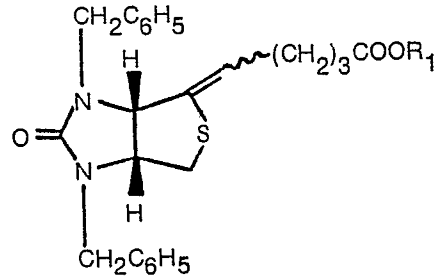

(III)

dans laquelle $R_1$ est comme défini précédemment.

2. Procédé selon la revendication 1, caractérisé en ce que $R_1$ est un atome d'hydrogène.

3. Procédé selon la revendication 1, caractérisé en ce que le composé (I) est dans une forme d.

4. Procédé selon la revendication 1, caractérisé en ce que la base est un composé de formule:

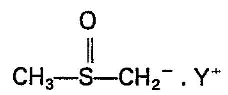

dans laquelle Y est un atome de lithium, un atome de sodium ou un atome de potassium.

5. Procédé selon la revendication 1, caractérisé en ce que le composé (III) est ensuite soumis à une réduction pour donner un composé de formule:

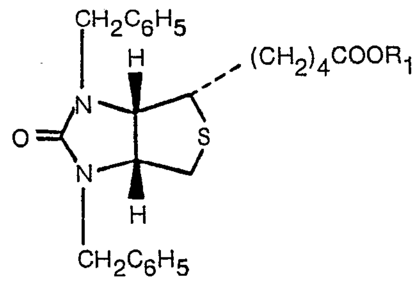

(IV)

dans laquelle $R_1$ est comme défini précédemment.

6. Procédé selon la revendication 5, caractérisé en ce que la réduction est mise en oeuvre par hydrogénation catalytique utilisant le carbone de palladium, l'oxyde de palladium ou un nickel résistant au soufre comme catalyseur.

7. Procédé selon la revendication 5, caractérisé en ce que le composé (IV) est ensuite soumis à une débenzylation pour donner un composé de formule:

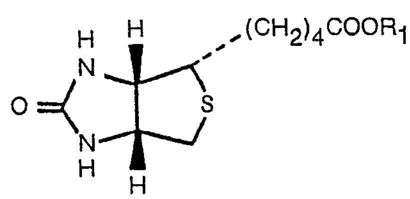

(V)

dans laquelle $R_1$ est comme défini précédemment.

8. Procédé selon la revendication 7, caractérisé en ce que la débenzylation est mise en oeuvre par traitement avec un acide méthanesulfonique.

9. Procédé selon la revendication 7, caractérisé en ce que le composé (IV) dans lequel $R_1$ est un groupe alkyle en $C_1$—$C_4$ est d'abord hydrolysé en composé (IV) dans lequel $R_1$ est un atome d'hydrogène et ce dernier est ensuite soumis à une débenzylation.

10. Procédé selon la revendication 7, caractérisé en ce que le composé (IV) dans lequel $R_1$ est un groupe alkyle en $C_1$—$C_4$ est d'abord soumis à une débenzylation pour donner le composé (V) dans lequel $R_1$ est un groupe alkyle en $C_1$—$C_4$ puis ce dernier est ensuite hydrolysé.

11. Procédé selon la revendication 7, caractérisé en ce que le composé (IV) dans lequel $R_1$ est un groupe alkyle en $C_1$—$C_4$ est soumis simultanément à une débenzylation et à une hydrolyse pour donner le composé (V) dans lequel $R_1$ est un atome d'hydrogène.

12. Procédé de préparation de la biotine ou de son ester qui comprend les étapes de (a) réaction du composé de formule:

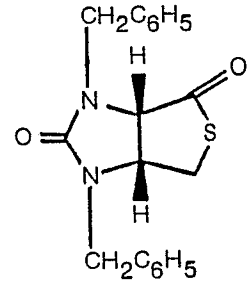

(I)

avec un composé de formule:

$$(C_6H_5)_3P^+(CH_2)_4COOR_1\ .\ X^- \qquad \text{(II)}$$

dans laquelle $R_1$ est un atome d'hydrogène ou un groupe alkyle en $C_1$—$C_4$ et X est un atome d'halogène en présence d'une base pour donner un composé de formule:

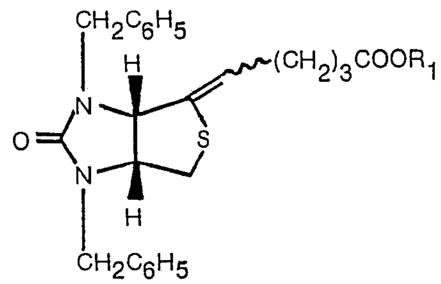

(III)

dans laquelle $R_1$ est comme défini précédemment, (b) soumission de ce dernier à une réduction pour donner un composé de formule:

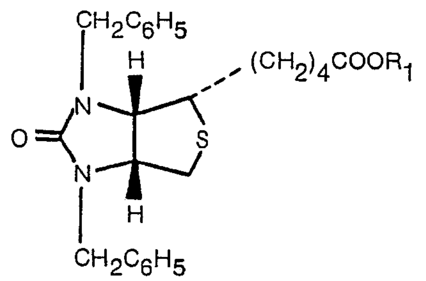

(IV)

dans laquelle $R_1$ est comme défini précédemment et (c) soumission de ce dernier à une débenzylation pour donner un composé de formule:

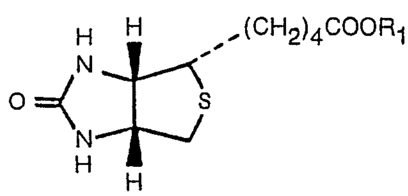

(V)

dans laquelle $R_1$ est défini comme précédemment.

13. Procédé selon la revendication 12, caractérisé en ce que le composé (IV) dans lequel $R_1$ est un groupe alkyle en $C_1$—$C_4$ est soumis à une débenzylation, accompagnée d'une première hydrolyse, hydrolyse simultanée ou hydrolyse ultérieure pour donner le composé (V) dans lequel $R_1$ est un atome d'hydrogène.